(19) Europäisches Patentamt
European Patent Office
Office européen des brevets

(11) **EP 2 143 801 A1**

(12) **EUROPEAN PATENT APPLICATION**
published in accordance with Art. 153(4) EPC

(43) Date of publication:
**13.01.2010 Bulletin 2010/02**

(51) Int Cl.:
*C12P 7/06* (2006.01)   *C12R 1/865* (2006.01)

(21) Application number: **08738950.8**

(22) Date of filing: **26.03.2008**

(86) International application number:
**PCT/JP2008/055765**

(87) International publication number:
**WO 2008/120643 (09.10.2008 Gazette 2008/41)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.03.2007 JP 2007095727**
**25.03.2008 JP 2008077762**
**25.03.2008 JP 2008077763**
**25.03.2008 JP 2008077765**

(71) Applicant: **Mitsui Engineering and Shipbuilding Co, Ltd.**
**Tokyo 104-8439 (JP)**

(72) Inventors:
• **TOHYAMA, Masayuki**
**Ichihara-shi**
**Chiba 290-8601 (JP)**
• **KATAGIRI, Manabu**
**Ichihara-shi**
**Chiba 290-8601 (JP)**
• **TAKAOKA, Kazue**
**Ichihara-shi**
**Chiba 290-8601 (JP)**

(74) Representative: **Gulde Hengelhaupt Ziebig & Schneider**
**Patentanwälte - Rechtsanwälte**
**Wallstraße 58/59**
**10179 Berlin (DE)**

(54) **METHOD FOR CONTINUOUSLY PRODUCING ALCOHOL**

(57) [Object]
The invention provides methods for continuous production of an alcohol that are capable of preventing the cell outflow to enhance the efficiency of contact between the alcohol raw material and the yeast, thereby fully utilizing the alcohol productivity of the yeast having flocculating and settling properties.

[Solution Means]
A method for continuous production of an alcohol including placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

the alcohol fermentation is conducted with an interface being formed; or cell concentration d1 and cell concentration d2 are measured at different vertical positions of an alcohol fermentation broth in the fermenter, and the liquid flow is controlled so that the ratio of d2 to d1 (d2/d1) is 1.7 or more and 6.6 or less; or cell concentration d1, cell concentration d2, and cell concentration d3 are measured at different vertical positions of the alcohol fermentation broth in the fermenter, and the liquid flow is controlled so that the ratio of d2 to d1 (d2/d1) is 1.7 or more, and the ratio of d3 to d1 (d3/d1) is 7.6 or less.

FIG. 1

**Description**

Technical Field

The present invention relates to methods for continuous production of an alcohol, and more particularly to methods for continuous production of an alcohol without cell recycle, using AM12 cells that exhibits a higher ethanol productivity than conventional yeasts.

Background Art

Patent Literature 1 discloses a method for producing an alcohol using a yeast strain *Saccharomyces cerevisiae* AM12 (hereinafter referred to as "strain AM12", as needed), and discloses that AM12 cells possess excellent alcohol productivity and alcohol tolerance, as well as flocculating and settling properties.
Patent Literature 1: Japanese Unexamined Patent Publication No. 59-135896

Disclosure of the Invention

Problems to be Solved by the Invention

In continuous production of an alcohol, an alcohol raw material is continuously fed into a fermenter, and alcoholic fermentation is conducted by yeast, while the resulting alcohol culture is withdrawn from the fermenter. In the continuous production of an alcohol without cell recycle, the cells in the alcohol culture are not returned to the fermenter.

It is preferable that the contents in the fermenter be completely mixed by agitation or the like, because the larger the number of contact times between the alcohol raw material and the yeast, the higher the reaction efficiency becomes. When complete mixing is performed, however, a large amount of yeast is removed together when the alcohol culture is withdrawn from the fermenter, causing the cell concentration in the fermenter to decrease.

If the amount of yeast growth is small relative to the amount of the withdrawn alcohol culture, the amount of the cell outflow cannot be compensated for. Therefore, the amount of the yeast cells present in the fermenter decreases, causing a substantial decrease in the number of contact times between the alcohol raw material and the yeast, resulting in a decrease in the efficiency of the alcohol-producing reaction.

On the other hand, if the contents in the fermenter are not completely agitated, the yeast having flocculating and settling properties will settle and deposit on the bottom of the fermenter. When the settling of the cells proceeds, the settling zone will change to a compaction zone after a lapse of a predetermined time, resulting in the formation of a layer zone (compaction zone) at the bottom. When an alcohol raw material is fed from the bottom of the fermenter, the alcohol raw material passes through the layer zone and come into contact with the yeast. However, the contact takes place in the compacted layer zone where the amount of yeast cells is in excess of the amount of the alcohol raw material; therefore, the efficiency of the reaction between the alcohol raw material and the yeast is extremely low.

The above-described problem is particularly noticeable with AM12 cells, because AM12 cells have a low cell growth ability, although they exhibit high alcohol productivity and alcohol tolerance.

The present inventors studied an approach of increasing the dilution rate to further increase the amount of ethanol produced in a method for continuous alcoholic fermentation. Consequently, they found that in the case of heretofore-used yeasts, the amount of ethanol produced is limited even if the dilution rate is increased in order to increase the amount of ethanol produced.

For this reason, the present inventors conducted a method for continuous alcoholic fermentation using AM12 cells that exhibit a higher ethanol productivity than conventional yeasts. Consequently, they found that when the dilution rate of the solution in the continuous reactor is increased in order to increase the ethanol productivity, the cells flow out of the reactor at once, and a high ethanol productivity cannot be maintained.

Accordingly, it is an object of the invention to provide methods for continuous production of an alcohol that are capable of maintaining a high ethanol productivity by defining an optimum relationship between the ethanol productivity of AM12 cells and the dilution rate.

It is another object of the invention to provide methods for continuous production of an alcohol that are capable of preventing the cell outflow to enhance the efficiency of contact between the alcohol raw material and the yeast, thereby fully utilizing the alcohol productivity of the yeast having flocculating and settling properties.

Other objects of the invention will become apparent from the following description.

Means for Solving the Problems

The above-described objects are solved by the inventions set forth below.

The invention as defined in claim 1 is a method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein the alcoholic fermentation is conducted with a cell interface being formed.

The invention as defined in claim 2 is the method according to claim 1, wherein the interface can be observed as a boundary of a layer with a high yeast cell concentration and a layer with a low yeast cell concentration that are formed in an alcohol fermentation broth in the fermenter.

The invention as defined in claim 3 is the method according to claim 1 or 2, wherein the yeast having flocculating and settling properties is *Saccharomyces cerevisiae* strain AM12.

The invention as defined in claim 4 is the method according to any one of claims 1 to 3, wherein the average yeast cell concentration in the fermenter is 20 to 40 g (dry weight)/L.

The invention as defined in claim 5 is the method according to any one of claims 1 to 4, wherein a yeast cell concentration above the interface is less than 14 g (dry weight)/L, a yeast cell concentration below the interface is 31 to 38 g (dry weight)/L.

The invention as defined in claim 6 is the method according to any one of claims 1 to 5, wherein the presence or absence of the interface is visually determined through an observation window formed on a side face of the fermenter.

The invention as defined in claim 7 is the method according to any one of claims 1 to 5, wherein the presence or absence of the interface is measured using a fermentation element and a light-receiving element provided above a liquid surface in the fermenter.

The invention as defined in claim 8 is the method according to any one of claims 1 to 5, wherein the presence or absence of the interface is detected using a plurality of turbidimeters provided in a height direction downward from the liquid surface of the alcohol fermentation broth in the fermenter.

The invention as defined in claim 9 is the method according to any one of claims 1 to 8, wherein the forced liquid flow is created in the lower portion of the fermenter principally by means of rotation of an agitator.

The invention as defined in claim 10 is a method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein cell concentration d1 and cell concentration d2 are measured at different vertical positions of an alcohol fermentation broth in the fermenter, and the liquid flow in the lower portion of the fermenter is controlled so that the ratio of the cell concentration d2 to the cell concentration d1 (d2/d1) is 1.7 or more and 6.6 or less.

The invention as defined in claim 11 is the method according to claim 10, wherein the cell concentration d1 and cell concentration d2 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.0 or more and 6.0 or less.

The invention as defined in claim 12 is the method according to claim 10 or 11, wherein the cell concentration d1 and cell concentration d2 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.3 or more and 5.6 or less.

The invention as defined in claim 13 is the method according to any one of claims 10 to 12, wherein the yeast having flocculating and settling properties is *Saccharomyces cerevisiae* strain AM12.

The invention as defined in claim 14 is the method according to any one of claims 10 to 13, wherein the average yeast cell concentration in the fermenter is 20 to 40 g (dry weight)/L.

The invention as defined in claim 15 is the method according to claim 10, 13, or 14, wherein the forced liquid flow is created in the lower portion of the fermenter principally by means of rotation of an agitator; and wherein the rotation of the agitator is controlled so that, when the ratio of the cell concentration d2 to the cell concentration d1 (d2/d1) is less than 1.7, the rotation of the agitator is ceased, or the number of revolutions is reduced; and when d2/d1 exceeds 6.6, the rotation of the agitator is started, or the number of revolutions is increased.

The invention as defined in claim 16 is the method according to any one of claims 10 to 15, wherein the cell concentrations are measured using a turbidimeter.

The invention as defined in claim 17 is a method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein cell concentration d1, cell concentration d2, and cell concentration d3 are measured at different vertical positions of an alcohol fermentation broth in the fermenter, and the liquid flow in the lower portion of the fermenter is controlled so that the ratio of the cell concentration d2 to the cell concentration d1 (d2/d1) is 1.7 or more, and the ratio of the cell concentration d3 to the cell concentration d1 (d3/d1) is 7.6 or less.

The invention as defined in claim 18 is the method according to claim 17, wherein the cell concentration d1, cell concentration d2, and cell concentration d3 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.0 or more, and the ratio of the cell concentration d3 to the cell concentration d1 (d3/d1) is 6.6 or less.

The invention as defined in claim 19 is the method according to claim 17, wherein the cell concentration d1, cell concentration d2, and cell concentration d3 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.3 or more, and the ratio of the cell concentration d3 to the cell concentration d1 (d3/d1) is 6.1 or less.

The invention as defined in claim 20 is the method according to any one of claims 17 to 19, wherein the yeast having flocculating and settling properties is *Saccharomyces cerevisiae* strain AM12.

The invention as defined in claim 21 is the method according to any one of claims 17 to 20, wherein the average yeast cell concentration in the fermenter is 20 to 40 g (dry weight)/L.

The invention as defined in claim 22 is the method according to claim 17, 20, or 21, wherein the forced liquid flow is created in the lower portion of the fermenter principally by means of rotation of an agitator; and wherein the rotation of the agitator is controlled so that, when the ratio of the cell concentration d2 to the cell concentration d1 (d2/d1) is less than 1.7, the rotation of the agitator in the fermenter is ceased, or the number of revolutions is reduced; and when the ratio of the cell concentration d3 to the cell concentration d1 (d3/d1) exceeds 7.6, the rotation of the agitator is started, or the number of revolutions is increased.

The invention as defined in claim 23 is the method according to any one of claims 17 to 22, wherein the cell concentrations are measured using a turbidimeter.

Effects of the Invention

In accordance with the invention, methods for continuous production of an alcohol are provided that are capable of maintaining a high ethanol productivity by defining an optimum relationship between the ethanol productivity of AM12 cells and the dilution rate.

Moreover, in accordance with the invention, methods for continuous production of an alcohol are provided that are capable of preventing the cell outflow to enhance the efficiency of contact between the alcohol raw material and the yeast, thereby fully utilizing the alcohol productivity of the yeast having flocculating and settling properties.

Brief Description of the Drawings

Fig. 1 is a schematic diagram of a continuous ethanol fermenter;

Fig. 2 is a graph showing the ethanol productivity of AM12 cells and the dilution rate;

Fig. 3 is a graph showing the relationship between the dilution rate and the steady-state concentration of AM12 cells in the fermenter;

Fig. 4 is a schematic diagram showing a preferable position in terms of height at which the interface is formed;

Fig. 5 is a schematic diagram showing a position of the interface at which the proportion of the layer (B) with a high yeast cell concentration relative to the volume V of the alcohol fermentation broth is preferable;

Fig. 6 is a schematic diagram of a fermenter having an interface observation window;

Fig. 7 is a schematic diagram showing an example of a fermenter usable in the invention;

Figs, 8 (A), (B), (C), and (D) are diagrams showing other examples of the shapes of the fermenter usable in the invention;

Fig. 9 is a flowchart showing one example of the control of the invention;

Fig. 10 is a schematic diagram of a control unit;

Fig. 11 is a schematic diagram showing another example of a fermenter usable in the invention;

Fig. 12 is a flowchart showing an example of the control of the invention;

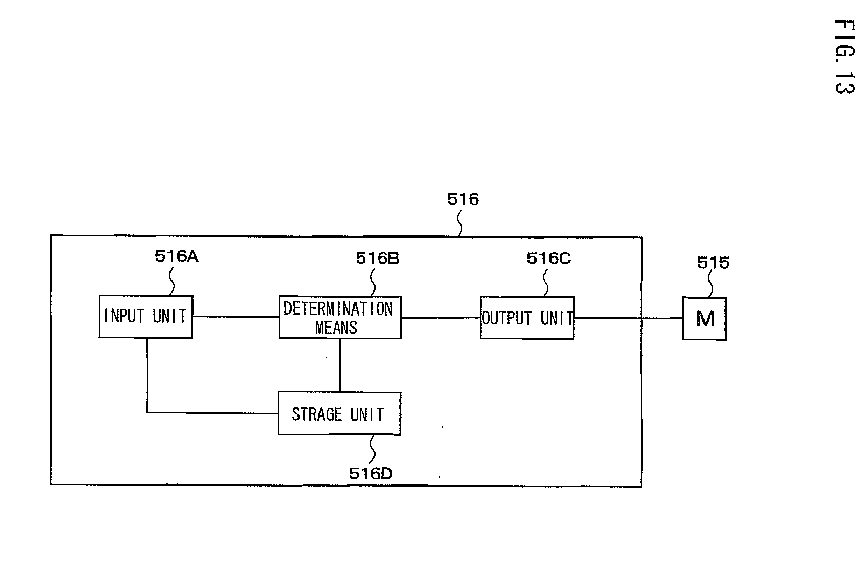

Fig. 13 is a schematic diagram of a control unit;

Fig. 14 is a graph showing the number of revolutions of an agitator and the cell concentration;

Fig. 15 shows the yeast cell concentration, glucose concentration, and ethanol concentration;

Fig. 16 is a graph showing the ethanol productivity and the dilution rate; and

Fig. 17 is a graph showing the number of revolutions of an agitator and the aeration rate.

Explanation of Reference Numerals

# EP 2 143 801 A1

1: Sugar Solution Feed Tank

10: Feed Pump

2: Fermenter

20: Ph Controller
21: Ph Meter
22: Ph Adjustment Pump
23: Tank
24: Temperature Controller
25: Thermometer
26: Cooling Pipe
27: DO Controller
28: DO Meter
29: Impeller
30: Alcohol Pump
301: Fermenter
302: Observation Window
310: Raw Material Inlet
311: Outlet
312: Agitator
401: Fermenter
410: Raw Material Inlet
411: Outlet
412: Densitometer
413: Densitometer
415: Agitator
415A: Agitation Motor
415B: Impeller
416: Control Unit
416A: Input Unit
416B: Determination Means
416C: Output Unit
416D: Storage Unit
501: Fermenter
510: Raw Material Inlet
511: Outlet
512: Densitometer
513: Densitometer
514: Densitometer
516: Control Unit
516A: Input Unit
516B: Determination Means
516C: Output Unit
516D: Storage Unit

Best Mode for Carrying Out the Invention

Embodiments of the present invention will hereinafter be described.

The methods for continuous production of an alcohol of the invention are methods including placing a yeast having flocculating and settling properties into a fermenter, and feeding an alcohol raw material to the fermenter to conduct alcoholic fermentation; and creating a forced liquid flow in a lower portion of the fermenter during the alcoholic fermentation.

The purpose of creating a forced liquid flow in the lower portion of a fermenter is to increase the number of contact times between the fermentation raw material and the yeast to increase the efficiency of the fermentation reaction. However, when a forced liquid flow is created in a fermenter by agitation or the like, the cells float upward and may flow out of the fermenter together with the fermentation broth. The yeast with flocculating and settling properties used in the invention inherently exhibits significant flocculating and settling properties, so that the cell concentration near the liquid surface in the fermenter becomes low, which inhibits the cell outflow from the fermenter.

The present inventors used a yeast with flocculating and settling properties in a method for continuous production of an alcohol, and adjusted the forced liquid flow in the lower portion of the fermenter. As a result, the cells floated upward by the liquid flow induced by agitation or the like, while they flocculated and settled due to the flocculating and settling properties of the yeast. Consequently, the boundary between these layers could be observed as an interface.

The inventors found that when the liquid flow in the lower portion of the fermenter is controlled based on the presence of this interface (this control is hereinafter referred to as "interface control", as needed), or when the liquid flow in the lower portion of the fermenter is controlled so that the ratio of cell concentrations measured at two or three different vertical positions in the fermenter falls within a specific range (this control is hereinafter referred to as "concentration control", as needed), the cell outflow can be prevented, and the alcohol productivity of the yeast with flocculating and settling properties can be fully exhibited.

The term "continuous production of an alcohol" as used herein means that a sugar solution, which is an alcohol raw material, is, for example, continuously fed into a fermenter, while the product is, for example, continuously withdrawn from the fermenter. Therefore, the alcohol raw material may be discontinuously fed as long as the amount of feed per unit time is constant.

In the invention, the yeast of the invention can maintain a high ethanol productivity when ethanol is continuously produced under conditions such that the dilution rate D ($h^{-1}$) is $0 < D \leq 0.3$, and the ethanol productivity J (g/L.h) is represented by $J = aD$, wherein the coefficient a is $82 \leq a \leq 108$. That is, the invention proposes the relationship between the ethanol productivity and the dilution rate.

To achieve a high ethanol productivity, it is preferred to perform the above-described interface control and concentration control. For example, when a cell interface is formed in the fermenter, the region below the interface having a high cell concentration is different from the compaction zone formed by flocculation and settling of the cells; the cell concentration below the interface and that above the interface are extremely different. With this interface being formed, the fermentation reaction of the alcohol raw material is promoted rather than inhibited.

In the continuous production of an alcohol of the invention, a small amount of cells may possibly flow out of the fermenter although the outflow of the cells from the fermenter is inhibited. However, any decrease in the cell concentration in the fermenter due to the cell outflow is prevented. In the invention, the amount of cell growth in the fermenter is larger than the amount of cell outflow, because a suitable balance of concentrations is maintained vertically in the fermenter; thus, the invention does not basically intend to constantly add the cells into the fermenter. However, the effects of the invention are by no means impaired by adding the cells as an inoculum, or by separating the small amount of cells that have flown out of the fermenter, and returning the cells into the fermenter.

The alcohol raw material used in the invention is preferably a raw material derived from grains. Preferable examples include those from rice, wheat, and potatos, because of their high sugar concentrations. In view of efficient saccharification reaction of the grain raw material, the sugar concentration is preferably 100 to 300 g/L, and more preferably 150 to 200 g/L, when calculated as glucose.

In view of an optimum fermentation temperature of the yeast, the aerobic fermentation temperature is preferably 40°C or less, and more preferably 30 to 35°C. The temperature may be controlled by employing a control means such as a heater or a cooler, but the means for controlling the temperature is not limited thereto.

In the invention, DO (Dissolved Oxygen Concentration) in the fermenter is preferably 0 to 3 ppm, and more preferably 0 to 0.1 ppm. Depending on the growth condition of the cells, DO may be zero. DO can be adjusted by increasing or decreasing the amount of air fed and the rotation speed of the impeller in the fermenter. For example, a method of interlocking a DO meter with a means for controlling the amount of air fed can be employed.

In view of an optimum pH of the yeast, the pH is preferably 3 to 7, and more preferably 3.5 to 5.0. The pH can be adjusted by employing a pH control system that adjusts the pH by continuously feeding a pH adjuster based on the data measured using a pH meter.

The yeast with flocculating and settling properties may be any yeast having settling properties such that an interface can be formed even during agitation. Yeast strain *Saccharomyces cerevisiae* AM12 or yeast strain *Saccharomyces cerevisiae* TJ1 is preferably used.

Both strains AM12 and TJ1 have been deposited with National Institute of Bioscience and Human Technology, Microorganism Research Institute (currently Advanced Industrial Science and Technology, International Patent Organism Dipositary (IPOD)) under the accession numbers 6749 and 6747, respectively, and are available therefrom.

Embodiments of the invention are hereinafter described with reference to the drawings.

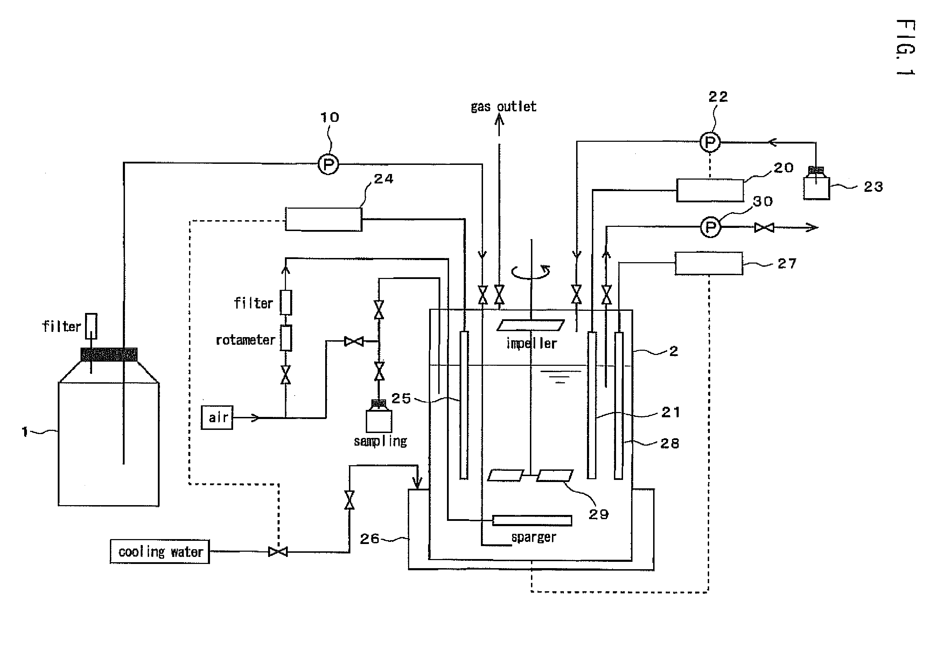

Referring to Fig. 1, reference numeral 1 denotes a sugar solution feed tank, and reference numeral 2 denotes a fermenter. A sugar solution is fed from the sugar solution feed tank 1 into the fermenter 2 using a feed pump 10.

The fermenter 2 has a pH controller 20. A measured value is input to the pH controller 20 from a pH meter 21. When the pH is not within the range of the control conditions described below, the pH controller 20 operates a pH adjustment pump 22, which then feeds 1N-HCL and 2N-NaOH to the fermenter 2 from the tank 23.

The fermenter 2 also has a temperature controller 24. A measured temperature is input to the temperature controller 24 from a thermometer 25. When the temperature exceeds the range of the control conditions described below, the temperature controller 24 adjusts the temperature by feeding cooling water to a cooling pipe 26 disposed around the periphery of the fermenter 2 for cooling, or by heating using a heater.

The fermenter further has a DO controller 27 and a DO meter 28 for controlling the dissolved oxygen (DO) concentration by aeration and by increasing or decreasing the rotation speed of an impeller 29 in the fermenter.

Reference numeral 30 denotes an alcohol pump for withdrawing the alcohol product, which is delivered to a purification process.

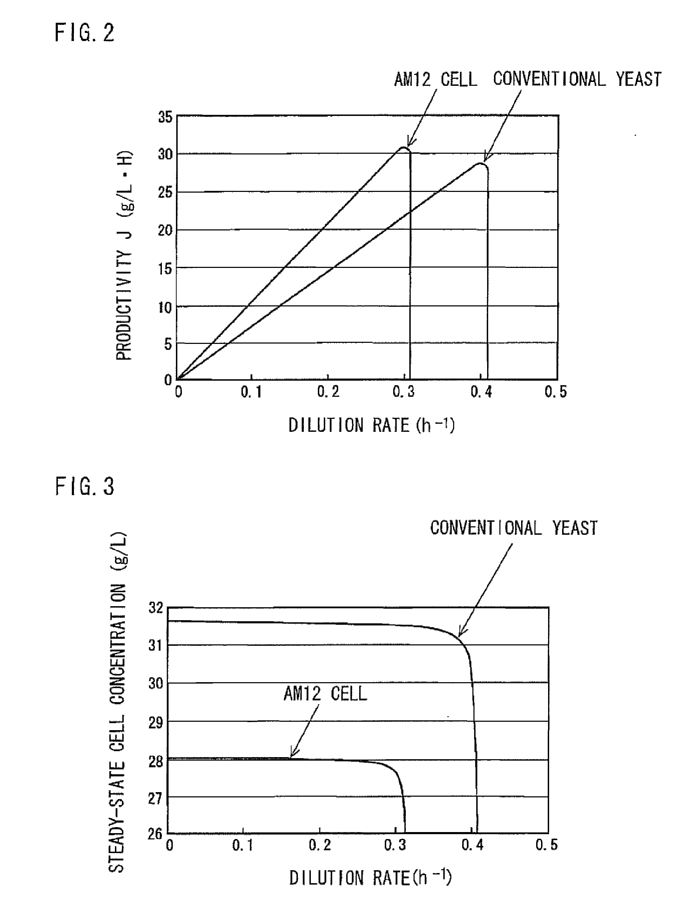

Unlike conventional yeasts, the yeast strain AM12 used in the invention exhibits an ethanol productivity superior to cell growth according to the reaction scheme (1) in microaerophilic culture (oxygen supply as low as about 1 ppm). This can be attributed to an extremely high ethanol productivity per cell (Fig. 2).

$$C_6H_{12}O_6 + N_2\text{-source} + O_2 \rightarrow \text{cell} + C_2H_5OH + CO_2$$

(microaerophilic conditions)

The continuous production of an alcohol without cell recycle has the advantage of being free of problems with contamination, leading to reduced facility costs. However, when the dilution rate is increased in order to obtain a high ethanol productivity, the AM12 cells having a low growth rate flow out of the reactor at once, causing the cell concentration in the fermenter 2 to decrease. Thus, a high ethanol productivity cannot be maintained.

Accordingly, in the invention, the relationship between the growth rate of AM12 cells in the fermenter and the dilution rate at which AM12 cells flow out of the fermenter was clarified to find a relationship between the dilution rate and the ethanol productivity of AM12 cells that can maintain a high ethanol productivity.

In the invention, the dilution rate D ($h^{-1}$) is $0 < D \leq 0.3$, and the ethanol productivity J (g/L·h) is represented by J = aD, wherein the coefficient a is $82 \leq a \leq 108$. The dilution rate D is a result of dividing the feed rate of the saccharified solution by the culture volume.

If the dilution rate D is 0.3 or more, the problem of cell outflow will occur; hence, the dilution rate D is preferably 0.1 to 0.3, and more preferably 0.14 to 0.18.

The cell concentration x (the average yeast cell concentration) is preferably 20 to 40 g/L, more preferably 25 to 33 g/L, and still more preferably 26 to 32 g/L. The cell concentration can be measured by measuring absorbance (turbidity).

The present inventors found that AM12 cells exhibit a very high ethanol productivity and ethanol tolerance; hence, with AM12 cells, the amount of ethanol produced can be shifted to a level that is clearly higher than that of general yeasts even at the same dilution rate. This is evident from Fig. 2, which shows the relationship between the dilution rate and the ethanol productivity of AM12 cells.

Fig. 3 shows the relationship between the dilution rate and the steady-state concentration of AM12 cells in the fermenter.

The coefficient a is preferably $82 \leq a \leq 108$, more preferably $86 \leq a \leq 106$, and still more preferably $88 \leq a \leq 105$. This range of the coefficient a cannot be satisfied by general yeasts.

Next, a preferred method of attaining the above-described dilution rate and ethanol productivity is described.

<Interface Control>

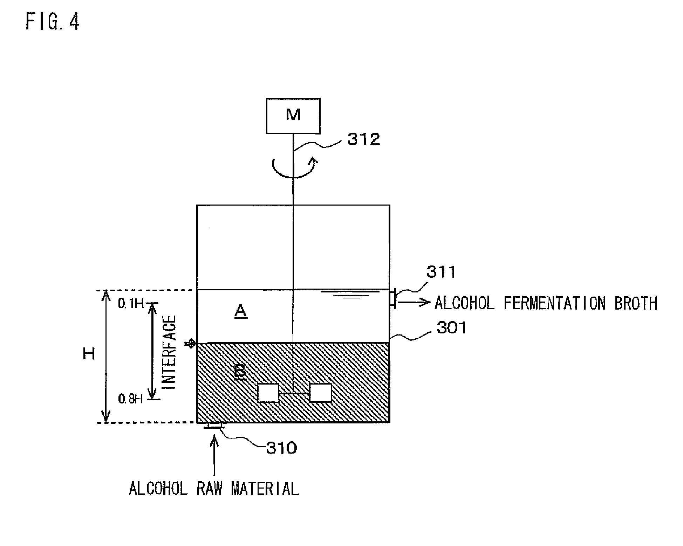

Fig. 4 shows an example of producing an alcohol with an interface being formed in the fermenter. In Fig. 4, reference numeral 301 denotes a fermenter, and an alcohol raw material is fed via a raw material inlet 310 at the bottom of the fermenter 301. The fermenter 301 contains a yeast with flocculating and settling properties.

When the alcohol raw material is, for example, continuously fed into the fermenter 301 via the raw material inlet 310, alcoholic fermentation is conducted by the yeast to produce an alcohol fermentation broth. The alcohol fermentation broth is withdrawn via an outlet 311. The location of the raw material inlet 310 is shown only by way of example, and the alcohol raw material may be fed by any method as long as it is fed via the bottom of the fermenter. Similarly, the alcohol fermentation broth can be withdrawn by any method as long as it is withdrawn via an upper portion of the fermenter.

An important feature of the invention is that alcoholic fermentation is conducted with an interface being formed in the alcohol fermentation broth in the fermenter 301, even when a liquid flow is being induced by agitation or the like.

The term "interface" means a linear boundary of the surface that is observed in the alcohol fermentation broth, and formed at the boundary between two layers that differ in turbidity (transparency) or hue. The linear boundary is observed when the fermenter is viewed horizontally, and may not be necessarily straight.

These layers are discernible as two layers that differ in turbidity (transparency) or hue, because they have dramatically different yeast cell concentrations in the alcohol fermentation broth.

For example, above the interface, the turbidity is low, and the color of the alcohol fermentation broth itself is observed, while below the interface is cloudy and has a high turbidity because a large amount of yeast cells are present. Thus, the difference between the layers A and B is clear, allowing the presence or absence of the interface to be visually observed.

The layer (A) above the interface is a layer with an extremely low concentration of yeast cells, and the layer (B) below the interface is a layer with a high concentration of yeast cells.

Cell concentrations can be quantitatively expressed as follows. The average yeast cell concentration in the alcohol fermentation broth in the fermenter, which is a cell concentration measured when the alcohol fermentation broth is in the form of a complete mixture, and is equal to the result of dividing the total amount of yeast cells (dry weight: g) in the fermenter by the amount of the alcohol fermentation broth (L) in the fermenter, is preferably 20 to 40 (dry weight) /L, more preferably 25 to 33 g (dry weight)/L, and still more preferably 26 to 32 g (dry weight)/L. Comparatively, the layer (A) with a low yeast cell concentration above the interface has a yeast cell concentration of preferably less than 14 g (dry weight)/L, more preferably less than 10 g (dry weight)/L, and still more preferably less than 7 g (dry weight)/L; and the layer (B) with a high yeast cell concentration below the interface has a yeast cell concentration of preferably 31 to 38 g (dry weight)/L, more preferably 32 to 37 g (dry weight)/L, and still more preferably 33 to 36 g(dry weight)/L.

It is important that the interface be formed below the outlet 311 for withdrawing the alcohol fermentation broth. This allows the layer with a low yeast cell concentration of the alcohol fermentation broth to be withdrawn from the fermenter, thereby inhibiting the outflow of yeast cells.

Due to their different yeast cell concentrations, the layer (B) with a high yeast cell concentration naturally has an alcohol productivity higher than that of the layer (A) with a low yeast cell concentration. Therefore, the higher the proportion of the layer (B) with a high yeast cell concentration that is present below the interface is, the higher the productivity becomes.

Specifically, the interface is formed in a region with a depth of 0.1 to 0.8 H (H: liquid surface height), preferably 0.2 to 0.6 H, and more preferably 0.2 to 0.5 H, downward from the liquid surface of the alcohol fermentation broth in the fermenter. Alternatively, the layer (B) with a high yeast cell concentration accounts for 20 to 90%, preferably 40 to 80%, and still more preferably 50 to 80%, of the volume V of the alcohol fermentation broth, and is preferably formed at the bottom side (in the lower portion) of the fermenter.

Fig. 4 shows a preferable position at which the interface is formed in the height direction.

In a fermenter having a constant cross-sectional area, such as a cylindrical fermenter, the position at which the interface is observed is defined within a region with a depth of 0.1 to 0.8 H (H: liquid surface height), preferably 0.2 to 0.6 H, and more preferably 0.2 to 0.5 H, downward from the liquid surface of the alcohol fermentation broth in the fermenter.

When the interface is formed in a region with a depth of 0.1 to 0.8 H, preferably 0.2 to 0.6 H, and more preferably 0.2 to 0.5 H, there is a good balance between agitation and settling, so that a high cell concentration in the fermenter 301 is maintained, and the efficiency of contact between the yeast cells and the alcohol raw material is also high, resulting in a high ethanol productivity.

When the position of the interface is above 0.1 H, the alcohol fermentation broth is nearly in the form of a complete mixture. This is undesirable because the cells easily flow out of the outlet 311 to cause the amount of the cells in the fermenter to decrease, which makes the alcohol productivity lower.

When the position of the interface is below 0.8 H, the cells in the fermenter begin to deposit on the bottom of the fermenter, forming a settling zone or a compaction zone. In the settling zone or compaction zone, yeast is present in an amount larger than the alcohol raw material, even though the alcohol raw material is being fed via the bottom of the fermenter. This is undesirable because the efficiency of the reaction between the alcohol raw material and the yeast is extremely low, which also makes the alcohol productivity lower.

The interface cannot be observed if, for example, the alcohol fermentation broth is completely mixed, or the amount of the cells in the fermenter is too small to discern a difference in concentration. Both of these cases are undesirable in carrying out the methods for the production of an alcohol of the invention.

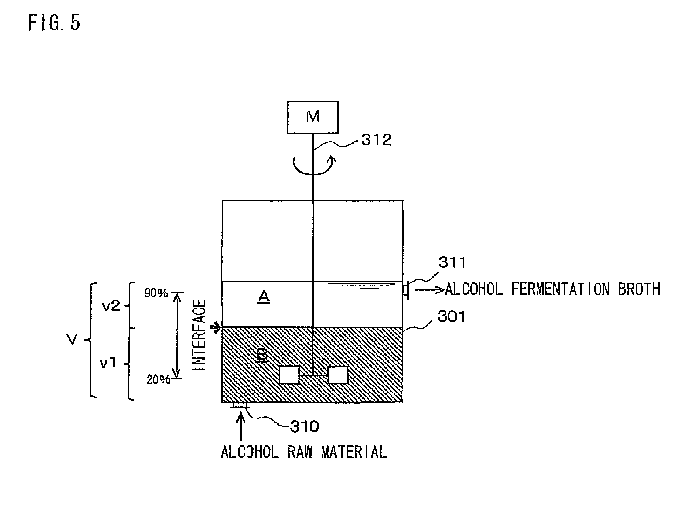

Fig. 5 is a schematic diagram showing a position of the interface at which the proportion of the layer (B) with a high yeast cell concentration relative to the volume V of the alcohol fermentation broth is preferable. Since the interface is formed at the boundary between the layer (A) with a low yeast cell concentration and the layer (B) with a high yeast cell concentration, the position of the interface can be defined by setting the proportion of the layer (B) with a high yeast cell concentration formed at the bottom side (in the lower portion) of the fermenter to 20 to 90%, preferably 40 to 80%, and more preferably 50 to 80%, relative to the volume V of the alcohol fermentation broth. Assuming that the volume of the layer (B) with a high yeast cell concentration is v1, and the volume of the layer (A) with a low yeast cell concentration is v2, V = v1 + v2.

When the proportion of the layer with a high yeast cell concentration is set in terms of volume, the method of the invention is also applicable to cases where the fermenter has a shape with a variable cross-sectional area, or where the position of the interface is difficult to define in terms of height.

In the invention, when the volume v1 of the layer (B) with a high yeast cell concentration accounts for more than 90% of the volume V of the alcohol fermentation broth, the alcohol fermentation broth is nearly in the form of a complete mixture. This is undesirable because the cells easily flow out of the outlet 311 to cause the amount of the cells in the fermenter to decrease, which makes the alcohol productivity lower.

Conversely, when the volume v1 of the layer (B) with a high yeast cell concentration is lower than 20% of the volume v of the alcohol fermentation broth, the cells in the fermenter begin to deposit on the bottom of the fermenter, forming a settling zone or a compaction zone. In the settling zone or compaction zone, yeast is present in an amount larger than the alcohol raw material, even though the alcohol raw material is being fed via the bottom of the fermenter. This is undesirable because the efficiency of the reaction between the alcohol raw material and the yeast is extremely low, which also makes the alcohol productivity lower.

In the invention, a means for creating a forced liquid flow in the lower portion of the fermenter is provided, in addition to the liquid flow created by feeding the alcohol fermentation broth. The forced liquid flow is preferably created principally by means of rotation of an agitator. Specifically, the forced liquid flow may be created by using an agitator alone, or by using an agitator and aeration in combination, but the use of an agitator alone is preferred in view of the convenience of control.

In the examples of Figs. 4 and 5, an agitator 312 with an impeller is shown as the means for agitation.

The position of the interface can be maintained by suitably varying the operating conditions for continuous fermentation, such as the intensity of agitation, so that the interface can be formed in the above-described preferable region.

The interface rises as the force of the liquid flow to cause the cells to flow upward increases; conversely, the interface begins to settle and fall down as the force of the liquid flow to cause the cells to flow upward decreases.

For example, the interface rises when the rotation of the agitator is increased.

The interface also rises when the amount of the alcohol raw material or the amount of the air fed is increased to cause an upward flow.

When the alcohol fermentation broth is in the form of a complete mixture, the rotation of the agitator may be ceased, or the rotation may be reduced to allow the formation of an interface.

The presence or absence of the interface can be visually observed because of the difference in hue or transparency between the layer (A) with a low yeast cell concentration and the layer (B) with a high yeast cell concentration.

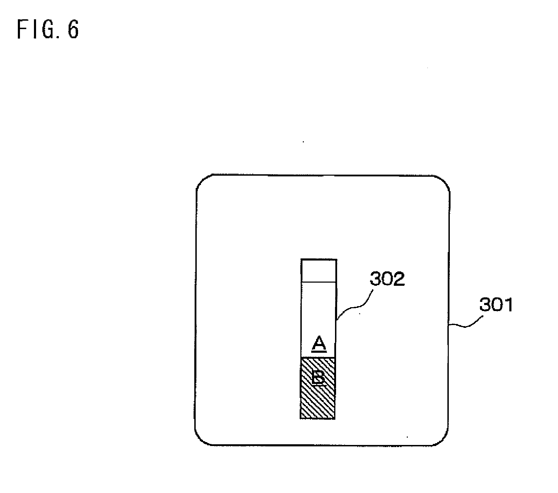

In the case of a fermenter that is not transparent, such as a stainless steel fermenter, the fermenter 301 may be provided with an observation window 302, as shown in Fig. 6, which allows one to abserve the presence or absence of the interface.

Alternatively, the presence or absence of the interface can be confirmed by measuring the cell concentration using two or more turbidimeters provided vertically at different positions downward from the liquid surface of the alcohol fermentation broth in the fermenter.

Still alternatively, the presence or absence of the interface can be measured by using a light-emitting element and a light-receiving element provided above the liquid surface in the fermenter. In this way, the light from the light-emitting element is reflected at the interface, and the reflected light is received by the light-receiving element, to determine the change in the amount of light.

<Concentration Control>

A first embodiment of the concentration control is next described referring to drawings.

In this embodiment, the liquid flow below the fermenter is controlled so that the ratio of the cell concentrations measured at two different vertical positions in the fermenter is within a specific range.

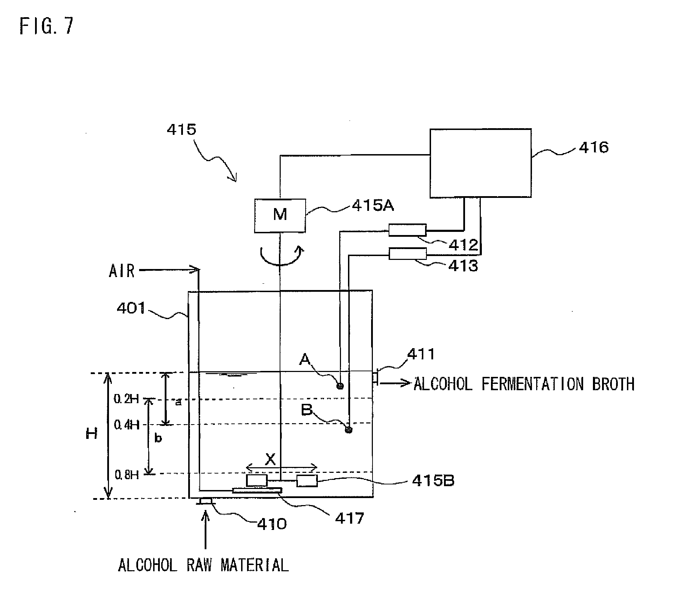

In Fig. 7, reference numeral 401 denotes a fermenter, and an alcohol raw material is fed via a raw material inlet 410 at the bottom of the fermenter 401. A yeast with flocculating and settling properties has been placed in the fermenter 401.

When an alcohol raw material is, for example, continuously fed into the fermenter 401 via the raw material inlet 410, alcoholic fermentation is conducted by the yeast to produce an alcohol fermentation broth. The alcohol fermentation broth is withdrawn via an outlet 411. The location of the raw material inlet 410 is shown only by way of example, and the alcohol raw material may be fed by any method as long as it is fed via the bottom of the fermenter. Similarly, the alcohol fermentation broth can be withdrawn by any method as long as it is withdrawn via an upper portion of the fermenter.

In the invention, the cell concentration d1 and cell concentration d2 are measured at different vertical positions of the alcohol fermentation broth in the fermenter.

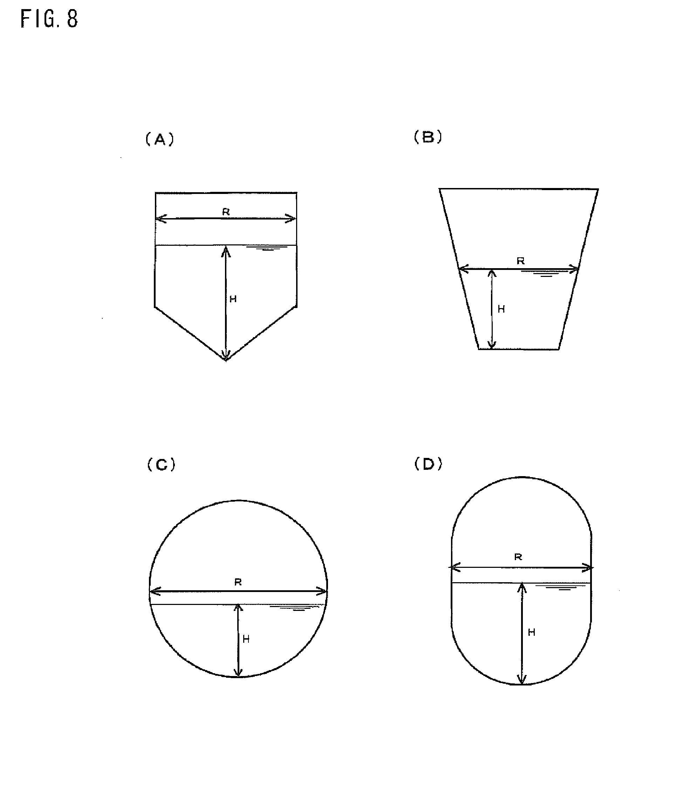

The different vertical positions can be various positions depending on the shape of the fermenter. Other than the cylindrical shape shown in Fig. 7, the fermenter may, for example, have the shapes shown in Figs. 8 (A) to (D).

For example, in the case of the cylindrical shape shown in Fig. 7, the cell concentration d1 is measured at cell concentration measurement point A provided in region (a) with a depth of 0.4 H (liquid surface height H) from the liquid surface. Therefore, a densitometer 412 is provided at this position. The cell concentration d2 is measured at cell concentration measurement point B provided in region (b) with a depth of 0.2 to 0.8 H. A densitometer 413 is provided at the position of this cell concentration measurement point for measurement. Examples of densitometers for measuring the cell concentrations include turbidimeters (the determination of absorbance). Note that the cell concentration measurement point A is always provided above the cell concentration measurement point B.

In order to univocally determine the concentration measurement positions in a fermenter having a shape other than the cylindrical shape shown in Fig. 8, the above-described depth regions in the cylindrical shape can be applied when the ratio of the liquid surface height (H) to the diameter (R) (H/R) is 0.5 to 2.0.

The diameter R is the maximum length of the cross section of a fermenter in the horizontal direction. In the case of a square shape, the diameter R represents the maximum length of the lengths of the diagonal lines, and the liquid surface height H represents a height from the liquid surface of the alcohol fermentation broth in the fermenter to the bottom surface of the fermenter.

In addition to the above-described method of defining the concentration measurement positions in terms of the depth from the liquid surface, the positions can be defined in terms of the volume of the alcohol fermentation broth in the fermenter 1.

For example, the cell concentration d1 is measured at cell concentration measurement point A provided in region (a) with a depth of 0.4 V (V represents the volume of the fermentation broth) from the liquid surface. The cell concentration d2 is measured at cell concentration measurement point B provided in region (b) with a depth of 0.2 to 0.8 V from the liquid surface.

In the invention, a means for creating a forced liquid flow in the lower portion of the fermenter is provided. The forced liquid flow is preferably created principally by means of rotation of an agitator. Specifically, the forced liquid flow may be created by using an agitator alone, or by using an agitator and aeration in combination, but the use of an agitator alone is preferred in view of the convenience of control.

Here, when the means for creating a forced liquid flow is an impeller, the region "the lower portion of the fermenter" where the impeller is located means that the impeller is located preferably below a depth of 0.5 H from the liquid surface, and more preferably below a depth of 0.8 H from the liquid surface, with respect to the liquid surface depth H of the fermenter.

Reference numeral 415 denotes an agitator, reference numeral 415A denotes an agitation motor, and reference numeral 415B denotes an impeller, which is preferably a plate-like paddle in the invention. In the embodiment shown in the figure, the impeller 415B is located below the depth region (b), and agitation is performed horizontally.

More specifically, when the fermenter is 126 mm in diameter, and has a liquid volume of 1000 ml (85 mm from the bottom of the fermenter), it is preferable that the center of an impeller with four plate blades (the maximum rotation diameter (X) of the impeller: 64 mm, the size of each blade: 15 x 12 mm) be positioned 77 mm below the liquid surface. The position "77 mm below the liquid surface" corresponds to the region of 0.9 H (H is the depth of the liquid surface) from the liquid surface.

The number of revolutions of the impeller is suitably determined within the range of preferably 30 to 150 rpm, more preferably 40 to 120 rpm, and still more preferably 50 to 100 rpm.

This number of revolutions is the numerical value determined using the above-described fermenter; hence, a preferable number of revolutions will vary under different conditions such as the fermenter and the like. Specifically, when the size of the fermenter, the diameter of the impeller, or the depth of the fermenter changes, the number of revolutions needs to be considered according to a scale-up or scale-down method. In scaling up or down, a number of revolutions that is determined from the intensity of agitation (power), which is calculated according to the calculation method described below, may be employed.

For reference, the power of the fermenter used in the Examples is determined.

Based on the equipment data of the fermenter (inside diameter: 0.126 m, depth of the liquid H: 0.085 m, blade diameter d: 0.064 m, blade width b: 0.012 m, agitation speed: 50 rpm, a turbin with four flat blades (no buffle)); and the physical data of the alcohol fermentation broth (density $\rho$: 1.062 (kg/m$^3$), viscosity $\mu$: 35.55 (Pa·s)), the number of revolutions n is determined (= 0.833s$^{-1}$), and the agitation Reynolds number Re is determined according to the following equation:

[Equation 1]

$$\text{Agitation Reynolds Number } Re \equiv \frac{d(nd)\rho}{\mu} = \frac{nd^2\rho}{\mu}$$

To determine the power number Np, which is a non-dimensional number with respect to the power necessary for agitation, the Nagata's equation (Equation 2) with respect to a two-blade paddle with a blade width of b' and without a buffle is modified based on its relationship with the agitation Reynolds number Re.

[Equation 2]

$$Np \equiv \frac{A}{Re} + B\left(\frac{10^3 + 1.2\,Re^{0.66}}{10^3 + 3.2\,Re^{0.66}}\right)^p \left(\frac{H}{D}\right)^{(0.35 + b'/D)}$$

**wherein:**

$$A = 14 + (b'/D)\{670(d/D - 0.6)^2 + 185\}$$

$$B = 10^{\{1.3 - 4(b'/D - 0.5)^2 - 1.14(d/D)\}}$$

$$p = 1.1 + 4(b'/D) - 2.5(d/D - 0.5)^2 - 7(b'/D)^4$$

When the number of blades $n_p$ is other than 2, N is estimated according to the equation above, based on a two-blade puddle whose product of the number of blades and the blade width, $n_p \times b$, is constant, i.e., b' = $(n_p b)/2$. In the case of a four-blade impeller (np=4), b' = $(n_p b)/2$ = 0.024 m; hence, according to the Nagata's equation (Equation 2), the power number in a low Re range and without a buffle is $N_p$ = 493487.8. Therefore, the power P necessary to conduct agitation at 50 rpm in this fermenter is determined to be 0.325655 W, according to Equation 3:

[Equation 3]

$$P = N_p \rho n^3 d^5$$

When the specification of the fermentation or impeller changes, the power can be calculated as above, and the number of revolutions can be thus determined.

Next, in the invention, the frequency of measuring the cell concentration at each cell concentration measurement point is preferably about once in 30 minutes to 1 hour, in order to perform suitable liquid flow control, for example, agitation control. The measured data are input to a control unit 416.

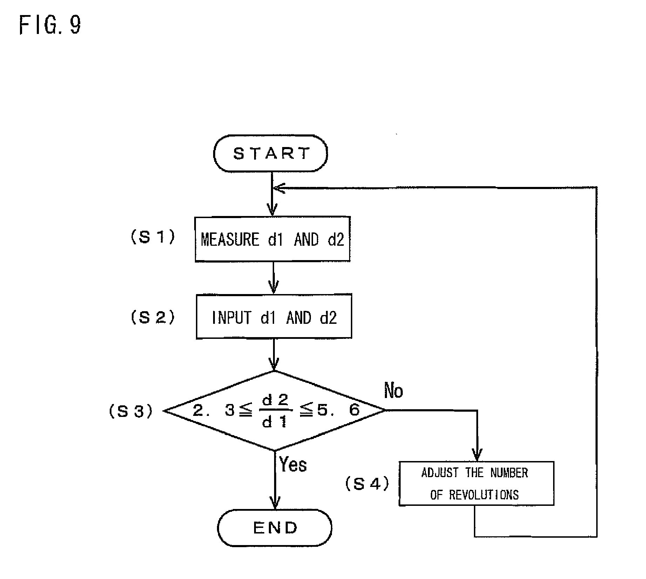

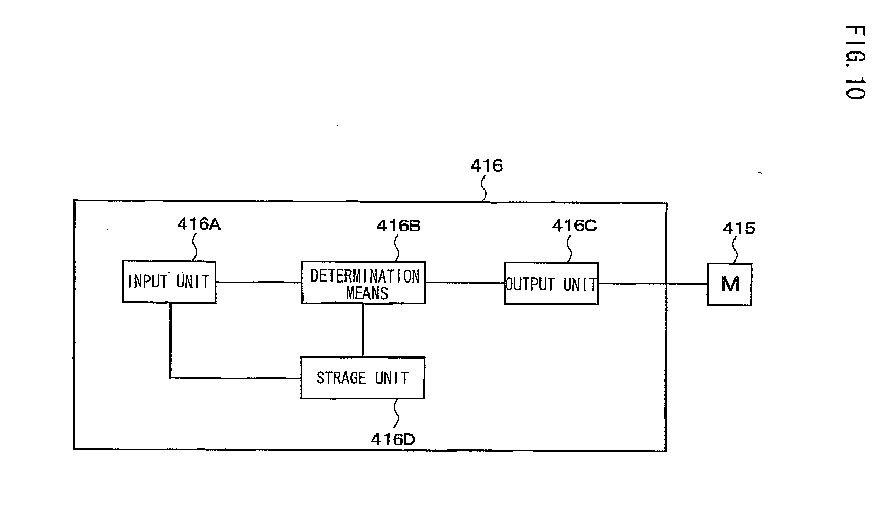

A preferred embodiment of the control method of the invention is hereinafter described referring to Figs. 9 and 10.

In this embodiment, the rotation of an agitator is controlled depending on the cell concentration d1 at cell concentration measurement point A and the cell concentration d2 at cell concentration measurement point B.

After the cell concentration d1 at cell concentration measurement point A and the cell concentration d2 at cell concentration measurement point B are measured (S1), the measured data are input to an input unit 416A of the control unit 416 (S2). The input measured data may preferably be stored in a storage unit 416D.

A determination means 416B makes a prescribed determination on the data that have been sent from the input unit 416A, and the determination result is sent to an output unit 416C for conversion to an output signal for control. The output signal is then sent to an agitator 415, whereupon the cessation or start of the rotation of the impeller is controlled.

The determination means 416B calculates the ratio of the cell concentration d2 at cell concentration measurement point B to the cell concentration d1 at cell concentration measurement point A (d2/d1), and determines whether the ratio is 1.7 or more and 6.6 or less (S3). The value of d2/d1 used for the control is preferably 2.0 or more and 6.0 or less, and more preferably 2.3 or more and 5.6 or less.

The example of Fig. 9 describes a representative case in which the threshold value of d2/d1 is set to 2.3 or more and 5.6 or less.

When the value of d2/d1 is 2.3 or more and 5.6 or less, the rotation of the agitator as measured is maintained.

When the value of d2/d1 is not 2.3 or more and 5.6 or less, the rotation of the agitator is adjusted (S4).

Specifically, when d2/d1 is less than 2.3, a signal to cease the rotation is sent from the output unit 416C to the agitator 415, thereby ceasing the rotation or reducing the number of revolutions of the impeller.

When d2/d1 is less than 2.3, due to excessively vigorous agitation, a large amount of cells are also present in the upper portion of the fermenter from which the alcohol fermentation broth is withdrawn, which causes the cells to easily flow out of the fementer. The settling of cells proceeds by ceasing the rotation or reducing the number of revolutions of the agitator 415, so that the cell concentration d1 decreases relative to the cell concentration d2, which increases the value of d2/d1.

Conversely, when d2/d1 exceeds 5.6, a signal to start the rotation is sent from the output unit 316C to the agitator 415, thereby starting the rotation or increasing the number of revolutions of the impeller.

In this embodiment, when d2/d1 exceeds the upper limit of the threshold value, it is believed that, due to excessively weak agitation, an unnecessarily large amount of cells have settled, and a layer zone (compaction zone) has begun to form. In this state, the efficiency of the reaction between the alcohol raw material and the yeast is poor. When the operation of the impeller is started, or the number of revolutions of the impeller is increased, the settling of cells can be solved by agitation, so that the cell concentration d1 increases relative to the cell concentration d2, which reduces the value of d2/d1.

After the rotation of the impeller has been adjusted, the concentrations d1 and d2 are measured again.

In order to confirm the effect of the rotation adjustment, it is preferable to conduct measurements after a lapse of 1 minute or more, and preferably 3 minutes or more, from the adjustment.

A second embodiment of the concentration control is next described referring to drawings.

In this embodiment, the liquid flow in the lower portion of the fermenter is controlled so that the ratios among the cell concentrations measured at three different vertical positions in the fermenter are within specific ranges. Features of the second embodiment are mainly described, and the description of the same apparatuses and control portions as described in the first embodiment is not repeated herein.

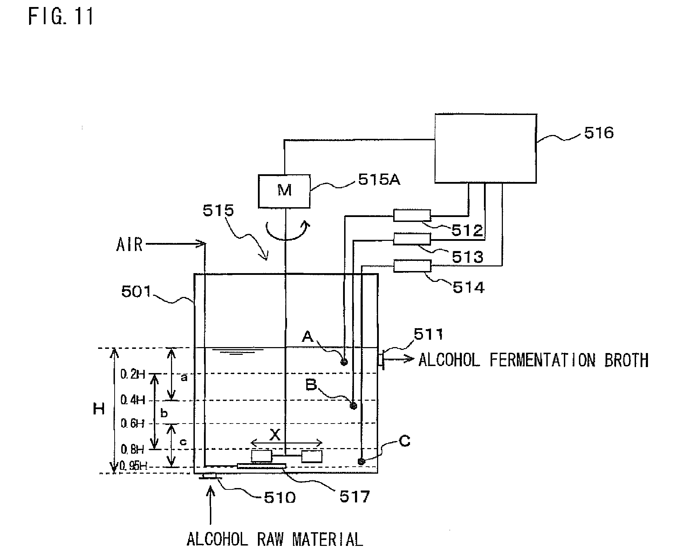

Referring to Fig. 11, when an alcohol raw material is, for example, continuously fed into the fermenter 501 via a raw material inlet 510, alcoholic fermentation is conducted by yeast to produce an alcohol fermentation broth. The alcohol fermentation broth is withdrawn via an outlet 511.

In the invention, cell concentration d1, cell concentration d2, and cell concentration d3 are measured at different vertical positions of the alcohol fermentation broth in the fermenter.

The different vertical positions can be various positions depending on the shape of the fermenter. Other than the cylindrical shape shown in Fig. 11, the fermenter may, for example, have the shapes shown in Figs. 8 (A) to (D).

For example, in the case of the cylindrical shape shown in Fig. 11, the cell concentration d1 is measured at cell concentration measurement point A provided in region (a) with a depth of 0.4 H (liquid surface height H) from the liquid surface. Therefore, a densitometer 512 is located at this position. The cell concentration d2 is measured at cell concentration measurement point B provided in region (b) with a depth of 0.2 to 0.8 H. A densitometer 513 is located at the position of this cell concentration measurement point for measurement. Further, the cell concentration d3 is measured at cell concentration measurement point C provided in region (c) with a depth of 0.6 to 0.95 H. A densitometer 514 is located at the position of this cell concentration measurement point for measurement.

Examples of densitometers for measuring the cell concentrations include turbidimeters (the determination of absorbance). Note that the cell concentration measurement point A is always provided above the cell concentration measurement point B, and the cell concentration measurement point B is always provided above the cell concentration measurement point C.

In order to univocally determine the concentration measurement positions in a fermenter having a shape other than the cylindrical shape shown in Fig. 11, the above-described depth regions in the cylindrical shape can be applied when the ratio of the liquid surface height (H) to the diameter (R) (H/R) is 0.5 to 2.0.

The diameter R is the maximum length of the cross section of a fermenter in the horizontal direction. In the case of a square shape, the diameter R represents the maximum length of the lengths of the diagonal lines, and the liquid surface height H represents a height from the liquid surface of the alcohol fermentation broth in the fermenter to the bottom surface of the fermenter.

In addition to the above-described method of defining the concentration measurement positions in terms of the depth from the liquid surface, the positions can be defined in terms of the volume of the alcohol fermentation broth in the fermenter 501.

For example, the cell concentration d1 is measured at cell concentration measurement point A provided in region (a) with a depth of 0.4 V (V represents the volume of the fermentation broth) from the liquid surface. The cell concentration d2 is measured at cell concentration measurement point B provided in region (b) with a depth of 0.2 to 0.8 V from the liquid surface. Further, the cell concentration d3 is measured at cell concentration measurement point C provided in region (c) with a depth of 0.6 to 0.95 V from the liquid surface.

A second embodiment of the control method is next described.

In this embodiment, the rotation of an agitator is controlled depending on the cell concentration d1 at cell concentration measurement point A, the cell concentration d2 at cell concentration measurement point B, and the cell concentration d3 at cell concentration measurement point C.

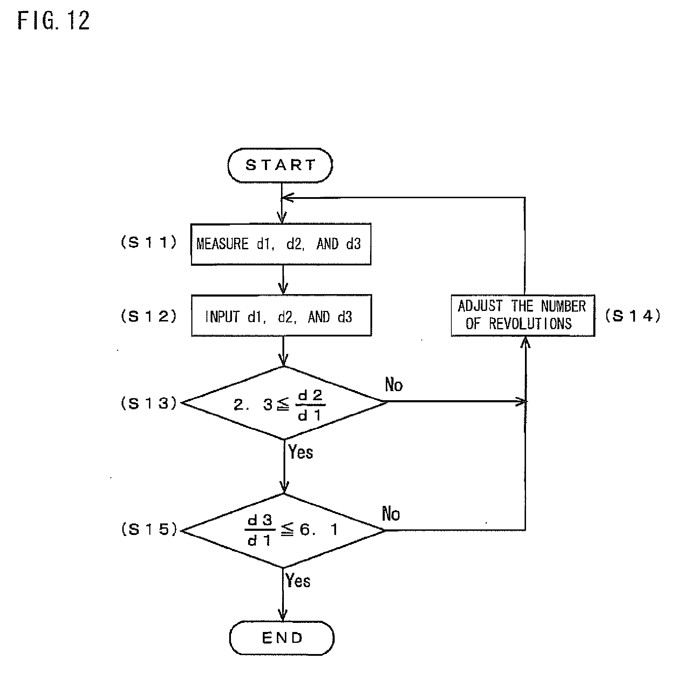

As shown in the flowchart of Fig. 12, after the cell concentration d1 at cell concentration measurement point A, the cell concentration d2 at cell concentration measurement point B, and the cell concentration d3 at cell concentration measurement point C are measured (S11) the measured data are input to an input unit 516A of a control unit 516 (S12). The input measured data may preferably be stored in a storage unit 516D.

A determination means 516B makes a predetermined determination on the data that have been sent from the input unit 516A, and the determination result is sent to an output unit 516C for conversion to an output signal for control. The output signal is then sent to an agitator 515, whereupon the cessation or start of the rotation of the impeller is controlled.

The determination means 516B calculates the ratios among the cell concentration d1, the cell concentration d2, and the cell concentration d3, and determines whether the ratios are in the range of threshold values (S13).

The threshold values used for the control are preferably such that d2/d1 is 1.7 or more, and d3/d1 is 7.6 or less; more preferably d2/d1 is 2.0 or more, and d3/d1 is 6.6 or less; and still more preferably d2/d1 is 2.3 or more, and d3/d1 is 6.1 or less.

The example of Fig. 12 describes a representative case in which the threshold value of d2/d1 is set to 2.3 or more, and d3/d1 is 6.1 or less.

When d2/d1 is 2.3 or more, the rotation of the agitator as measured is maintained. When d2/d1 is not 2.3 or more, the rotation of the agitator is adjusted (S14). Specifically, the rotation of the impeller is ceased, or the number of revolutions of the impeller is reduced.

The determination means 516B subsequently calculates the ratio of the cell concentration d3 to the cell concentration d1 (d3/d1), and determines whether the ratio is 6.1 or less (S15).

When the value of d3/d1 is 6.1 or less, the rotation of the agitator as measured is maintained.

When the value of d3/d1 is not 6.1 or less, the rotation of the agitator is adjusted (S14). Specifically, the rotation of the impeller is started, or the number of revolutions of the impeller is increased.

After the rotation of the impeller has been adjusted, the concentrations d1, d2, and d3 are measured again.

The control of the rotation of the agitator in S14 includes ceasing the rotation of the impeller, or reducing the number of revolutions of the impeller; and starting the rotation of the impeller, or increasing the number of revolutions of the impeller.

When d2/d1 is less than 1.7, due to excessively vigorous agitation, a large amount of cells are also present in the upper portion of the fermenter from which the alcohol fermentation broth is withdrawn, which causes the cells to easily flow out of the fementer.

The settling of cells proceeds by ceasing the rotation or reducing the number of revolutions of the agitator 515, so that the cell concentration d1 decreases relative to the cell concentration d2, which increases the value of d2/d1.

Conversely, when d2/d1 exceeds 7.6, a signal to start the rotation is sent from the output unit 516C to the agitator 515, thereby starting the rotation or increasing the number of revolutions of the impeller.

In this embodiment, when d2/d1 exceeds the upper limit of the threshold value, it is believed that, due to excessively weak agitation, an unnecessarily large amount of cells have settled, and a layer zone (compaction zone) has begun to form. In this state, the efficiency of the reaction between the alcohol raw material and the yeast is poor. When the operation of the impeller is started, or the number of revolutions of the impeller is increased, the settling of cells can be solved by agitation, so that the cell concentration d1 increases relative to the cell concentration d3, which reduces the value of d3/d1.

In order to confirm the effect of the rotation adjustment, it is preferable to conduct measurements after a lapse of 1 minute or more, and preferably 3 minutes or more, from the adjustment.

<u>EXAMPLE</u>

Examples of the present invention are described below; however, the invention is not limited by these Examples.

Example 1

<Preparation of Yeast Inoculum Culture>

10 ml of YPD medium (1% yeast extract, 2% peptone, 5% glucose, pH 5.0) was placed in a 200 ml flask and sterilized; the flask was subsequently inoculated with each yeast colony stored in yeast storage plates using a platinum loop, and incubated for 20 hours under aerobic conditions at 30°C and 200 rpm, to restore the yeast (AM12 cells) to the culturable state.

A 250 ml flask containing 100 ml of sterile YPD medium was inoculated with 10 ml of the restored yeast culture. The yeast was incubated for 20 hours under aerobic conditions at 30°C and 200 rpm, to further grow the yeast inoculum in the logarithmic growth phase.

<Continuous Fermentation with Jar fermenter>

110 ml of the above-described yeast inoculum culture was inoculated into a jar fermenter containing 900 ml of sterile CSL medium for start-up (1% corn steep liquor (CSL), 0.1% $KH_2PO_4$, 0.05% $MgSO_4 \cdot 7H_2O$, 0.01% $CaCl_2 \cdot H_2O$, 10% glucose, pH 5.0).

The yeast culture was inoculated using a feed pump to prevent exposure to the outside air.

The operating conditions of the jar fermenter were as follows.

pH: 5.0 (adjusted with 2N NaOH and HCl)

Aeration: 0.5 to 1.5 vvm (aeration was provided by passing air from a compressor via a sterile filter)

Agitation Speed: 50 to 150 rpm

Temperature: 30°C

The culture was sampled from the fermenter after 24 and 48 hours from the start of the fermentation, and the glucose concentration was measured to confirm that the fermentation broth had a sugar concentration of 5 g/L.

Sterile CSL medium for continuous production (1% CSL, 0.1% $KH_2PO_4$, 0.05% $MgSO_4 \cdot 7H_2O$, 0.01% $CaCl_2 \cdot H_2O$, 20% glucose, pH 5.0) was continuously fed into the jar fermenter, while the culture (alcohol fermentation broth) was withdrawn at the same rate as the medium fed (alcohol raw material), with the rotation of an agitator being adjusted so that an interface is formed. Continuous fermentation was thus started (the start of continuous fermentation).

A sample of the contents of the fermenter was taken every other day, and analyzed.

The yeast growth, the glucose consumption, and the amount of ethanol produced were analyzed according to the following methods.

The yeast growth was measured based on turbidity (absorbance: $OD_{600\,nm}$) to determine the concentration using a conversion factor to the cell concentration ($0.37 \times OD_{600\,nm}$).

The glucose consumption was measured using a glucose kit (Glucose Test C Wako; Wako Pure Chemical Ind. Ltd.). Change over time in ethanol production was measured by analyzing the ethanol concentration in the culture (Shimadzu GC-2014, detector FID).

Ethanol production was conducted under these conditions at a dilution rate of 0.1, using AM12 cells. As a result, the ethanol productivity was 9.7 g.L/h. Substituting this value into J = aD yielded a = 97.

Example 2

Ethanol production was conducted as in Example 1, except that the dilution rate was 0.14. As a result, the ethanol productivity was 14.4 g·L/h. Substituting this value into J = aD yielded a = 102.85.

Example 3

Ethanol production was conducted as in Example 1, except that the dilution rate was 0.18. As a result, the ethanol productivity was 17.7 g.L/h. Substituting this value into J = aD yielded a = 98.3.

Comparative Example 1

Ethanol production was conducted as in Example 1, except that the dilution rate was 0.1, using a conventional yeast (*Saccharomyces cerevisiae* strain (JCM7255)). As a result, the ethanol productivity was 7.4 g.L/h. Substituting this value into J = aD yielded a = 0.1, which was outside the range of the invention.

Comparative Example 2

Ethanol production was conducted as in Comparative Example 1, except that the dilution rate was 0.14. As a result, the ethanol productivity was 10.35 g·L/h. Substituting this value into J = aD yielded a = 0.14, which was outside the range of the invention.

Comparative Example 3

Ethanol production was conducted as in Comparative Example 1, except that the dilution rate was 0.18. As a result, the ethanol productivity was 13.3 g.L/h. Substituting this value into J = aD yielded a = 0.18, which was outside the range of the invention.

The above results revealed that ethanol productivity that satisfies the value a within the range of the invention was not obtained using the conventional yeast.

Example 4 (Interface Control)

<Continuous Fermentation Using the Fermenter Shown in Fig. 4>

An alcohol raw material (glucose) was fed into the fermenter while the fermentation broth (culture) was withdrawn at the same rate as the raw material fed. Continuous fermentation was thus conducted without cell recycle. In the following experiments, the AM12 yeast cells present in the fermenter may also be simply referred to as the cells.

The fermenter had the structure shown in Fig. 4. The fermenter was 126 mm in diameter, had a liquid volume of 1000 mL (about 85 mm from the bottom of the fermenter), and was equipped with an impeller whose number of revolutions can be varied (an impeller with four plate blades; the maximum rotation diameter of the impeller: 64 mm; the size of each blade: 15 x 12 mm).

The fermenter operating conditions except for the dilution rate and the number of revolutions of the agitator were as follows: temperature: 30°C, aeration rate: 0.15 vvm, sugar concentration in the alcohol raw material: 200 g/L.

Continuous fermentation was conducted at a dilution rate of 0.18 and a number of revolutions of the agitator of 50 rpm. An interface was formed at 0.42 H. The ethanol productivity was 17.8 g/L/h.

Example 5

Continuous fermentation was conducted at a dilution rate of 0.14 and a number of revolutions of the agitator of 150 rpm. An interface was formed at 0.23 H. The ethanol productivity was 12.2 g/L/h.

Comparative Example 4

Continuous fermentation was conducted at a dilution rate of 0.18 and a number of revolutions of the agitator of 200 rpm. As a result, the alcohol fermentation broth was in the form of a complete mixture, and the cell outflow occurred to significantly reduce the ethanol productivity.

Comparative Example 5

Continuous fermentation was conducted at a dilution rate of 0.18, and a number of revolutions of the agitator of 10 rpm. An interface was formed at 0.95 H. The ethanol productivity was 10 g/L/h.

Example 6 (Concentration Control at Two Points)

<Continuous Fermentation Using the Fermenter Shown in Fig. 7>

An alcohol raw material (glucose) was fed into the fermenter while the fermentation broth (culture) was withdrawn at the same rate as the raw material fed. Continuous fermentation was thus conducted using AM12 cells without cell recycle. In the following experiments, the AM12 yeast cells present in the fermenter may also be simply referred to as the cells.

The fermenter was 126 mm in diameter, and had a liquid volume of 1000 mL (85 mm from the bottom of the fermenter).

The fermenter operating conditions were as follows: temperature: 30°C, pH 5, sugar concentration of the alcohol raw material calculated as glucose: 200 g/L.

<Experiment 1>

This experiment examined the relationship between the number of revolutions of an agitator in the fermenter and the cell concentrations d1, d2 at cell concentration measurement points A, B, which are control factors in controlling the agitation in the fermenter.

The cell concentration measurement point A was set at 10 mm below the liquid surface (0.11 H), and the cell concentration measurement point B was set at 50 mm below the liquid surface (0.58 H).

An agitator whose number of revolutions can be varied was located so that the center of the impeller (an impeller with four plate blades; the maximum rotation diameter of the impeller: 64 mm, the size of each blade: 15 x 12 mm) was positioned 77 mm below the liquid surface (0.91 H).

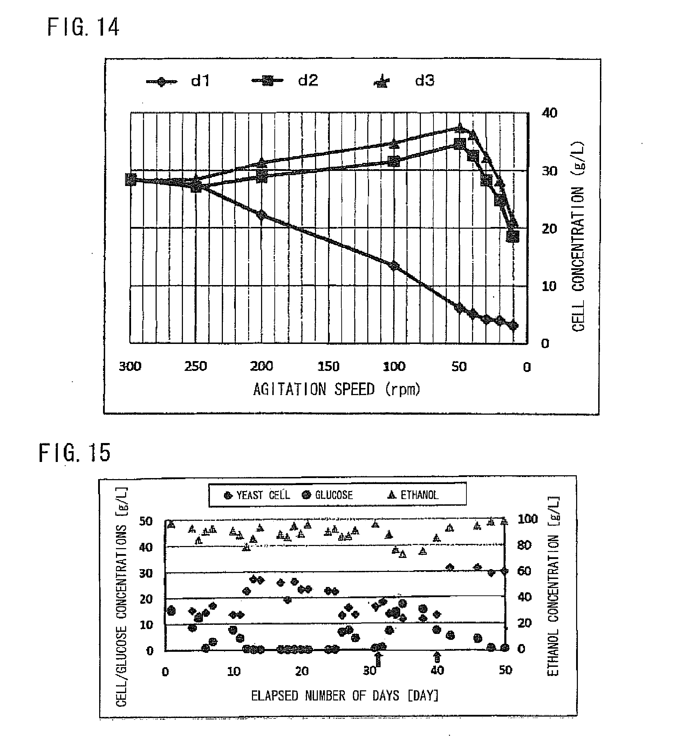

The results are shown in Fig. 14.

As shown in Fig. 14, the initial number of revolutions was set to 300 rpm, and the number of revolutions was thereafter reduced. The cell concentration at the cell concentration measurement point A began to decrease at 250 rpm, and decreased to about 6.2 g/L at 50 rpm. This shows that, at 50 rpm, the cell concentration from the liquid surface to 0.2 H is extremely small, with substantially no possibility of cell outflow.

It was found that the cell concentration at the cell concentration measurement point B shows little decrease even when the number of revolutions is reduced to 50 rpm. At less than 50 rpm, the cell concentration d2 begins to decrease.

In order to produce a portion with a low yeast cell concentration and a portion with a high yeast cell concentration by attaining a balance between flocculation and mixing to thereby prevent the yeast cells from flowing out of the fermenter, and promote efficient contact between the alcohol raw material and the yeast, an efficient range of the number of revolutions was assumed to be the range wherein the value of d2/d1 is large, and concentration d2 is high, without forming a layer zone where the efficiency of reaction (contact) between the alcohol raw material and the yeast is poor (i.e., the range wherein the curves representing the cell concentrations in Fig. 14 show peaks); accordingly, the concentration ratios (d2/d1) in the range of 30 to 150 rpm were determined based on Fig. 14.

As a result, the values of d2/d1 at 30, 40, 50, 100, 120, and 150 rpm were 6.6, 6.0, 5.6, 2.3, 2.0, and 1.7, respectively.

Therefore, the minimum value of d2/d1 was 1.7, and the maximum value was 6.6.

Based on this finding, in the invention, the minimum value d2/d1 = 1.7 and the maximum value d2/d1 = 6.6 were employed as threshold values of the control factors of an agitator.

<Experiment 2>

This experiment examined how the control of the number of revolutions of an agitator influences the ethanol productivity, using d2/d1 = 2.3 and d2/d1 = 5.6 as threshold values.

Continuous production of ethanol was conducted by feeding an alcohol raw material with a sugar concentration of 200 g/L (calculated as glucose) into the same fermenter as that of Experiment 1.

The positions of the cell concentration measurement points were the same as those of Experiment 1.

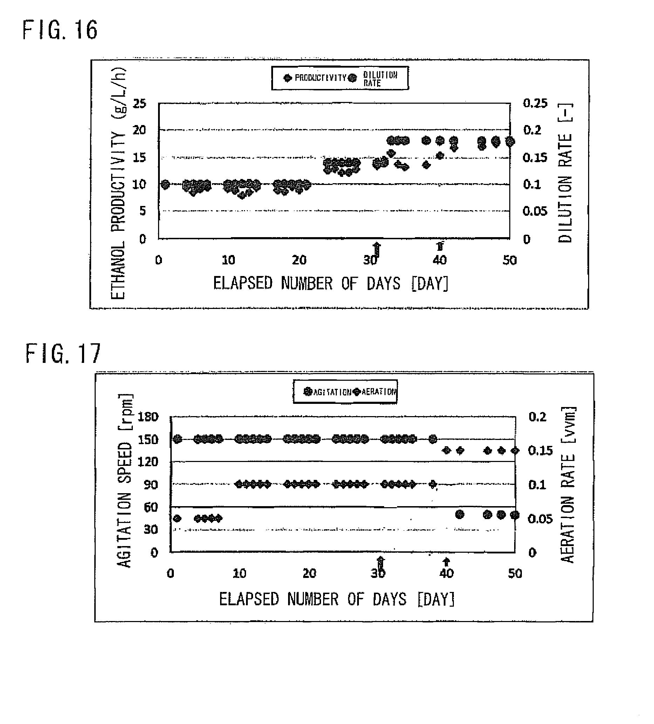

Fig. 15 is a graph showing the average yeast cell concentration in the fermenter, and the glucose concentration and ethanol concentration in the withdrawn alcohol fermentation broth. Fig. 16 is a graph showing the ethanol productivity and the dilution rate. Fig. 17 is a graph showing the number of revolutions during agitation and the aeration rate.

In order to increase the ethanol productivity, the dilution rate D was increased to match the cell growth, i.e., was 0.1 from the start of the continuous production to day 22, was 0.14 from day 22 to day 33, and was 0.18 from day 33 to day 50. At day 33 when the dilution rate was increased to 0.18, the average yeast cell concentration (by the squares in Fig. 15) dropped below 20 g/L.

This is because, when the dilution rate was increased to 0.18, the agitation with an agitator at the initial 150 rpm became too vigorous, causing the cell to float up into the upper layer and flow out.

Further, the ethanol concentration (by the triangles in Fig. 15) decreased, and the glucose concentration (by the circles in Fig. 15; the alcohol raw material that was not converted into ethanol) increased, resulting in a poor ethanol productivity.

Here, according to the invention, the number of revolutions was adjusted from day 40.

Since d2/d1 was less than 2.3, the number of revolutions was controlled to decrease; as a result, the average yeast cell concentration on day 41 was 30 g/L or more. Consequently, although the actual average ethanol concentration before the agitation control was 75.2 g/L, it increased to 93.9 g/L after the agitation control, resulting in an improved ethanol productivity (Table 1). Additionally, when the value of d2/d1 increased to more than 5.6 after continued operation at a reduced number of revolutions, the number of revolutions was controlled to increase.

[Table 1]

| Dilution Rate D (h$^{-1}$) | Average Ethanol Concentration (g/L) | Maximum Ethanol Productivity (g/L/hr) |
|---|---|---|
| 0.1 | 90.2 | 9.7 |
| 0.14 | 92.5 | 14.4 |
| 0.18 (Before Agitation Control) | 75.2 | 13.5 |
| 0.18 (After Agitation Control) | 93.9 | 16.9 |

Since the fermenter of this embodiment was cylindrical, the cell concentration measurement point B at a depth point of 0.58 H was in the region (b) with a depth of 0.2 to 0.8 V from the liquid surface, and the cell concentration measurement point A at a depth point of 0.11 H was in the region (a) with a depth of 0.4 V from the liquid surface (V represents the volume of the fermentation broth).

Example 7

(Concentration Control at Three Points)

<Continuous Fermentation Using the Fermenter Shown in Fig. 11>

An alcohol raw material (glucose) was fed into the fermenter while the fermentation broth (culture) was withdrawn at the same rate as the raw material fed. Continuous fermentation was thus conducted using AM12 cells without cell recycle. In the following experiments, the AM12 yeast cells present in the fermenter may also be simply referred to as the cells.

The fermenter was 126 mm in diameter, had a liquid volume of 1000 mL, and had a height (H) of 85 mm from the bottom of the fermenter to the liquid surface.

The fermenter operating conditions were as follows: temperature: 30°C, pH 5, sugar concentration of the alcohol raw material calculated as glucose: 200 g/L.

<Experiment 1>

This experiment examined the relationship between the number of revolutions of an agitator in the fermenter and cell concentrations d1, d2, d3 at cell concentration measurement points A, B, C, which are control factors in controlling the agitation in the fermenter.

The cell concentration measurement point A was set at 10 mm below the liquid surface (0.11 H), the cell concentration measurement point B was set at 50 mm below the liquid surface (0.58 H), and the cell concentration measurement point C was set at 80 mm below the liquid surface (0.94 H).

An agitator whose number of revolutions can be varied was located so that the center of the impeller (an impeller with four plate blades; the maximum rotation diameter of the impeller: 64 mm, the size of each blade: 15 x 12 mm) was positioned 77 mm below the liquid surface (0.91 H).

The results are shown in Fig. 14.

As shown in Fig. 14, the initial number of revolutions was set to 300 rpm, and the number of revolutions was thereafter reduced. The cell concentration at the cell concentration measurement point A began to decrease at 250 rpm, and decreased to about 6.2 g/L at 50 rpm. This shows that, at 50 rpm, the cell concentration from the liquid surface to 0.2 H is extremely small, with substantially no possibility of cell outflow.

It was found that the cell concentrations at the cell concentration measurement points B and C shows little decrease even when the number of revolutions is reduced to 50 rpm. It was found that the cell concentrations d2 and d3 begin to decrease at 50 rpm or less, and the cell concentrations at the cell concentration measurement points B and C shown in Fig. 14 shift in a very similar manner.

In order to produce a portion with a low yeast cell concentration and a portion with a high yeast cell concentration by attaining a balance between flocculation and mixing to thereby prevent the yeast cells from flowing out of the fermenter, and promote efficient contact between the alcohol raw material and the yeast, an efficient range of the number of revolutions was assumed to be the range wherein the values of d2/d1 and d3/d1 are large, and concentrations d2 and d3 are high, without forming a layer zone where the efficiency of reaction (contact) between the alcohol raw material and the yeast is poor (i.e., the range wherein the curves representing the cell concentrations in Fig. 14 show peaks); accordingly, the concentration ratios in the range of 30 to 150 rpm were determined.

As a result, the values of d2/d1 at 30, 40, 50, 100, 120, and 150 rpm were 6.6, 6.0, 5.6, 2.3, 2.0, and 1.7, respectively.

The values of d3/d1 at 30, 40, 50, 100, 120, and 150 rpm were 7.6, 6.6, 6.1, 2.4, 2.2, and 1.8, respectively.

Therefore, the minimum value of d2/d1 was 1.7, and the maximum value of d3/d1 was 7.6.

Based on this finding, in the invention, the minimum value d2/d1 = 1.7 and the maximum value d3/d1 = 7.6 were employed as threshold values of the control factors of an agitator.

<Experiment 2>

This experiment examined how the control of the number of revolutions of an agitator influences the ethanol productivity, using d2/d1 = 2.3 and d3/d1 = 6.1 as threshold values.

Continuous production of ethanol was conducted by feeding an alcohol raw material with a sugar concentration of 200 g/L (calculated as glucose) into the same fermenter as that of Experiment 1.

The positions of the cell concentration measurement points were the same as those of Experiment 1.

Fig. 15 is a graph showing the average yeast cell concentration in the fermenter, and the glucose concentration and ethanol concentration in the withdrawn alcohol fermentation broth. Fig. 16 is a graph showing the ethanol productivity and the dilution rate. Fig. 17 is a graph showing the number of revolutions during agitation and the aeration rate.

In order to increase the ethanol productivity, the dilution rate D was increased to match the cell growth, i.e., was 0.1 from the start of the continuous production to day 22, was 0.14 from day 22 to day 33, and was 0.18 from day 33 to day 50. At day 33 when the dilution rate was increased to 0.18, the average yeast cell concentration (by the squares in Fig. 15) dropped below 20 g/L.

This is because, when the dilution rate was increased to 0.18, the agitation with an agitator at the initial 150 rpm became too vigorous, causing the cell to float up into the upper layer and flow out.

Further, the ethanol concentration (by the triangles in Fig. 15) decreased, and the glucose concentration (by the circles in Fig. 15; the alcohol raw material that was not converted into ethanol) increased, resulting in a poor ethanol productivity.

Here, according to the invention, the number of revolutions was adjusted from day 40.

Since d2/d1 was less than 2.3, the number of revolutions was controlled to decrease; as a result, the average yeast cell concentration on day 41 was 30 g/L or more. Consequently, although the actual average ethanol concentration before the agitation control was 75.2 g/L, it increased to 93.9 g/L after the agitation control, resulting in an improved ethanol productivity (see Table 1 above). Additionally, when the value of d3/d1 increased to more than 6.1 after continued operation at a reduced number of revolutions, the number of revolutions was controlled to increase.

Since the fermenter of this embodiment was cylindrical, the cell concentration measurement point A at a depth point of 0.11 H was in the region (a) with a depth of 0.4 V from the liquid surface (V represents the volume of the fermentation broth); the cell concentration measurement point B at a depth point of 0.58 H was in the region (b) with a depth of 0.2 to 0.8 V from the liquid surface; and the cell concentration measurement point C at a depth point of 0.91 H was in the region (c) with a depth of 0.6 to 0.95 V from the liquid surface.

**Claims**

**1.** A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

the alcoholic fermentation is conducted with a cell interface being formed.

**2.** The method according to claim 1, wherein the interface can be observed as a boundary of a layer with a high yeast cell concentration and a layer with a low yeast cell concentration that are formed in an alcohol fermentation broth in the fermenter.

3. The method according to claim 1 or 2, wherein the yeast having flocculating and settling properties is *Saccharomyces cerevisiae* strain AM12.

4. The method according to any one of claims 1 to 3, wherein the average yeast cell concentration in the fermenter is 20 to 40 g (dry weight)/L.

5. The method according to any one of claims 1 to 4, wherein a yeast cell concentration above the interface is less than 14 g (dry weight)/L, a yeast cell concentration below the interface is 31 to 38 g (dry weight)/L.

6. The method according to any one of claims 1 to 5, wherein the presence or absence of the interface is visually determined through an observation window formed on a side face of the fermenter.

7. The method according to any one of claims 1 to 5, wherein the presence or absence of the interface is measured using a fermentation element and a light-receiving element provided above a liquid surface in the fermenter.

8. The method according to any one of claims 1 to 5, wherein the presence or absence of the interface is detected using a plurality of turbidimeters provided in a height direction downward from the liquid surface of the alcohol fermentation broth in the fermenter.

9. The method according to any one of claims 1 to 8, wherein the forced liquid flow is created in the lower portion of the fermenter principally by means of rotation of an agitator.

10. A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

    cell concentration d1 and cell concentration d2 are measured at different vertical positions of an alcohol fermentation broth in the fermenter, and the liquid flow in the lower portion of the fermenter is controlled so that the ratio of the cell concentration d2 to the cell concentration d1 (d2/d1) is 1.7 or more and 6.6 or less.

11. The method according to claim 10, wherein the cell concentration d1 and cell concentration d2 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.0 or more and 6.0 or less.

12. The method according to claim 10 or 11, wherein the cell concentration d1 and cell concentration d2 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.3 or more and 5.6 or less.

13. The method according to any one of claims 10 to 12, wherein the yeast having flocculating and settling properties is *Saccharomyces cerevisiae* strain AM12.

14. The method according to any one of claims 10 to 13, wherein the average yeast cell concentration in the fermenter is 20 to 40 g (dry weight)/L.

15. The method according to claim 10, 13, or 14, wherein the forced liquid flow is created in the lower portion of the fermenter principally by means of rotation of an agitator; and wherein:

    the rotation of the agitator is controlled so that, when the ratio of the cell concentration d2 to the cell concentration d1 (d2/d1) is less than 1.7, the rotation of the agitator is ceased, or the number of revolutions is reduced; and when d2/d1 exceeds 6.6, the rotation of the agitator is started, or the number of revolutions is increased.

16. The method according to any one of claims 10 to 15, wherein the cell concentrations are measured using a turbidimeter.

17. A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

cell concentration d1, cell concentration d2, and cell concentration d3 are measured at different vertical positions of an alcohol fermentation broth in the fermenter, and the liquid flow in the lower portion of the fermenter is controlled so that the ratio of the cell concentration d2 to the cell concentration d1 (d2/d1) is 1.7 or more, and the ratio of the cell concentration d3 to the cell concentration d1 (d3/d1) is 7.6 or less.

18. The method according to claim 17, wherein the cell concentration d1, cell concentration d2, and cell concentration d3 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.0 or more, and the ratio of the cell concentration d3 to the cell concentration d1 (d3/d1) is 6.6 or less.

19. The method according to claim 17, wherein the cell concentration d1, cell concentration d2, and cell concentration d3 are measured at different vertical positions of the alcohol fermentation broth in the fermenter, and the liquid flow in the lower portion of the fermenter is controlled so that the ratio of the cell concentration d2 to the cell concentration d1 (d2/d1) is 2.3 or more, and the ratio of the cell concentration d3 to the cell concentration d1 (d3/d1) is 6.1 or less.

20. The method according to any one of claims 17 to 19, wherein the yeast having flocculating and settling properties is *Saccharomyces cerevisiae* strain AM12.

21. The method according to any one of claims 17 to 20, wherein the average yeast cell concentration in the fermenter is 20 to 40 g (dry weight)/L.

22. The method according to claim 17, 20, or 21, wherein the forced liquid flow is created in the lower portion of the fermenter principally by means of rotation of an agitator; and wherein:

the rotation of the agitator is controlled so that, when the ratio of the cell concentration d2 to the cell concentration d1 (d2/d1) is less than 1.7, the rotation of the agitator in the fermenter is ceased, or the number of revolutions is reduced; and
when the ratio of the cell concentration d3 to the cell concentration d1 (d3/d1) exceeds 7.6, the rotation of the agitator is started, or the number of revolutions is increased.

23. The method according to any one of claims 17 to 22, wherein the cell concentrations are measured using a turbidimeter.

**Amended claims under Art. 19.1 PCT**

1. A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

the alcoholic fermentation is conducted with a cell interface being formed.

2. The method according to claim 1, wherein the interface can be observed as a boundary of a layer with a high yeast cell concentration and a layer with a low yeast cell concentration that are formed in an alcohol fermentation broth in the fermenter.

3. The method according to claim 1 or 2, wherein the yeast having flocculating and settling properties is *Saccharomyces cerevisiae* strain AM12.

4. The method according to any one of claims 1 to 3, wherein the average yeast cell concentration in the fermenter is 20 to 40 g (dry weight)/L.

5. The method according to any one of claims 1 to 4, wherein a yeast cell concentration above the interface is less than 14 g (dry weight)/L, a yeast cell concentration below the interface is 31 to 38 g (dry weight)/L.

6. The method according to any one of claims 1 to 5, wherein the presence or absence of the interface is visually determined through an observation window formed on a side face of the fermenter.

**7.** Amended)
The method according to any one of claims 1 to 5, wherein the presence or absence of the interface is measured using a light-emitting element and a light-receiving element provided above a liquid surface of the fermenter.

**8.** The method according to any one of claims 1 to 5, wherein the presence or absence of the interface is detected using a plurality of turbidimeters provided in a height direction downward from the liquid surface of the alcohol fermentation broth in the fermenter.

**9.** The method according to any one of claims 1 to 8, wherein the forced liquid flow is created in the lower portion of the fermenter principally by means of rotation of an agitator.

**10.** Cancelled)

**11.** Amended)
A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

cell concentration d1 is measured at cell concentration measurement point A provided in region (a) with a depth of 0.4 H (liquid surface height H) from a liquid surface of an alcohol fermentation broth in the fermenter, and cell concentration d2 is measured at cell concentration measurement point B provided in region (b) with a depth of 0.2 to 0.8 H; and
the liquid flow in the lower portion of the fermenter is controlled so that the ratio of the cell concentration d2 to the cell concentration d1 (d2/d1) is 2.3 or more and 5.6 or less.

**12.** Amended)
A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

cell concentration d1 is measured at cell concentration measurement point A provided in region (a) with a depth of 0.4 V (V represents the volume of a fermentation broth) from a liquid surface of an alcohol fermentation broth in the fermenter, and cell concentration d2 is measured at cell concentration measurement point B provided in region (b) with a depth of 0.2 to 0.8 V from the liquid surface; and
the liquid flow in the lower portion of the fermenter is controlled so that the ratio of the cell concentration d2 to the cell concentration d1 (d2/d1) is 2.3 or more and 5.6 or less.

**13.** Amended)
The method according to claim 11 or 12, wherein the yeast having flocculating and settling properties is *Saccharomyces cerevisiae* strain AM12.

**14.** Amended)
The method according to any one of claims 11 to 13, wherein the average yeast cell concentration in the fermenter is 20 to 40 g (dry weight)/L.

**15.** Amended)
The method according to claims 11 to 14, wherein the forced liquid flow is created in the lower portion of the fermenter principally by means of rotation of an agitator; and wherein:

the rotation of the agitator is controlled so that, when the ratio of the cell concentration d2 to the cell concentration d1 (d2/d1) is less than 2.3, the rotation of the agitator is ceased, or the number of revolutions is reduced; and when d2/d1 exceeds 5.6, the rotation of the agitator is started, or the number of revolutions is increased.

**16.** Amended)
The method according to any one of claims 11 to 15, wherein the cell concentrations are measured using a turbidimeter.

**17.** Cancelled)

**18.** Amended)
A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

cell concentration d1 is measured at cell concentration measurement point A provided in region (a) with a depth of 0.4 H (liquid surface height H) from a liquid surface of an alcohol fermentation broth in the fermenter, cell concentration d2 is measured at cell concentration measurement point B provided in region (b) with a depth of 0.2 to 0.8 H, and cell concentration d3 is measured at cell concentration measurement point C provided in region (c) with a depth of 0.6 to 0.95 H; and
the liquid flow in the lower portion of the fermenter is controlled so that the ratio of the cell concentration d2 to the cell concentration d1 (d2/d1) is 2.3 or more, and the ratio of the cell concentration d3 to the cell concentration d1 (d3/d1) is 6.1 or less.

**19.** Amended)
A method for continuous production of an alcohol comprising placing a yeast having flocculating and settling properties into a fermenter, and feeding an alcohol raw material to the fermenter to conduct alcoholic fermentation; and creating a forced liquid flow in a lower portion of the fermenter during the alcoholic fermentation; wherein:

cell concentration d1 is measured at cell concentration measurement point A provided in region (a) with a depth of 0.4 V (V represents the volume of a fermentation broth) from a liquid surface of an alcohol fermentation broth in the fermenter, cell concentration d2 is measured at cell concentration measurement point B provided in region (b) with a depth of 0.2 to 0.8 V from the liquid surface, and cell concentration d3 is measured at cell concentration measurement point C provided in region (c) with a depth of 0.6 to 0.95 V; and
the liquid flow in the lower portion of the fermenter is controlled so that the ratio of the cell concentration d2 to the cell concentration d1 (d2/d1) is 2.3 or more, and the ratio of the cell concentration d3 to the cell concentration d1 (d3/d1) is 6.1 or less.

**20.** Amended)
The method according to claim 18 or 19, wherein the yeast having flocculating and settling properties is *Saccharomyces cerevisiae* strain AM12.

**21.** Amended)
The method according to any one of claims 18 to 20, wherein the average yeast cell concentration in the fermenter is 20 to 40 g (dry weight)/L.

**22.** Amended)
The method according to any one of claims 18 to 21, wherein the forced liquid flow is created in the lower portion of the fermenter principally by means of rotation of an agitator; and wherein:

the rotation of the agitator is controlled so that, when the ratio of the cell concentration d1 to the cell concentration d2 (d2/d1) is less than 2.3, the rotation of the agitator in the fermenter is ceased, or the rotation speed is reduced; and
when the ratio of the cell concentration d1 to the cell concentration d3 (d3/d1) exceeds 6.1, the rotation of the agitator is started, or the rotation speed is increased.

**23.** Amended)
The method according to any one of claims 18 to 22, wherein the cell concentrations are measured using a turbidimeter.

FIG. 1

gas outlet

impeller

sparger

sampling

filter

rotameter

air

cooling water

filter

# FIG. 2

AM12 CELL    CONVENTIONAL YEAST

Axis labels: PRODUCTIVITY J (g/L·H) vs DILUTION RATE (h⁻¹)

# FIG. 3

CONVENTIONAL YEAST

AM12 CELL

Axis labels: STEADY-STATE CELL CONCENTRATION (g/L) vs DILUTION RATE (h⁻¹)

FIG. 4

M

~312

311

→ ALCOHOL FERMENTATION BROTH

301

0.1H

INTERFACE

A

H

B

0.8H

~310

ALCOHOL RAW MATERIAL

FIG. 5

FIG. 6

FIG. 7

AIR →

ALCOHOL FERMENTATION BROTH

ALCOHOL RAW MATERIAL

FIG. 8

(A)

(B)

(C)

(D)

FIG. 9

```
              ┌─────────────┐
              │   START     │
              └──────┬──────┘
                     │◄──────────────────────┐
                     ▼                        │
(S1)    ┌─────────────────────────┐          │
        │  MEASURE d1 AND d2       │          │
        └────────────┬────────────┘          │
                     ▼                        │
(S2)    ┌─────────────────────────┐          │
        │   INPUT d1 AND d2        │          │
        └────────────┬────────────┘          │
                     ▼                        │
                    ╱╲                No      │
(S3)          ╱           ╲  ───────────┐     │
            ╱  2.3 ≦ d2/d1 ≦ 5.6 ╲      │     │
              ╲           ╱             │     │
                    ╲╱                  ▼     │
                  Yes │         ┌──────────────────┐
                      ▼    (S4) │ ADJUST THE NUMBER │
              ┌─────────────┐   │  OF REVOLUTIONS   │──┘
              │    END      │   └──────────────────┘
              └─────────────┘
```

The condition in (S3): $2.3 \leqq \dfrac{d2}{d1} \leqq 5.6$

FIG. 10

EP 2 143 801 A1

416

416A
INPUT UNIT

416B
DETERMINATION
MEANS

416C
OUTPUT UNIT

415
M

STRAGE UNIT

416D

FIG. 11

AIR

515

501

0.2H
0.4H
0.6H
0.8H
0.95H

H

a

b

c

X

A

B

C

M — 515A

516

512
513
514

511

ALCOHOL FERMENTATION BROTH

510     517

ALCOHOL RAW MATERIAL

FIG. 12

START

(S 1 1) | MEASURE d1, d2, AND d3 |

(S 1 2) | INPUT d1, d2, AND d3 |

(S 1 3) $2.3 \leqq \dfrac{d2}{d1}$ — No → ADJUST THE NUMBER OF REVOLUTIONS (S 1 4)

Yes

(S 1 5) $\dfrac{d3}{d1} \leqq 6.1$ — No

Yes

END

FIG. 13

FIG. 14

FIG. 15

FIG. 16

FIG. 17

## INTERNATIONAL SEARCH REPORT

| International application No. |
|---|
| PCT/JP2008/055765 |

A. CLASSIFICATION OF SUBJECT MATTER
*C12P7/06*(2006.01)i, *C12R1/865*(2006.01)n

According to International Patent Classification (IPC) or to both national classification and IPC

B. FIELDS SEARCHED

Minimum documentation searched (classification system followed by classification symbols)
C12P7/06, C12R1/865

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)
BIOSIS/MEDLINE/WPIDS(STN), CAplus(STN), JSTPlus(JDreamII),
JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X/A | Wieczorek A. and Michalski H., Continuous ethanol production by flocculating yeast in the fluidized bed bioreactor., FEMS Microbiology Reviews, 1994, 14(1), p.69-74 | 1-11,13-18, 20-23/12,19 |
| A | JP 59-135896 A (Mitsui Engineering & Shipbuilding Co., Ltd.), 04 August, 1984 (04.08.84), & PH 18793 A | 1-23 |
| A | Ryoichi NISHIDA, "Saibo Yugokabu Kobo Ethanol Hakko Tokusei", Kagaku Keizai, 1985, 32(8), pages 26 to 32 | 1-23 |
| A | Kaihatsu Gijutsu Honbu, "Shin Kobo ni yoru Ethanol Hakko Gijutsu", Mitsui Zosen Technical Review, 1986, No.128, pages 42 to 47 | 1-23 |

[X]  Further documents are listed in the continuation of Box C.          [ ]  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 May, 2008 (15.05.08) | 27 May, 2008 (27.05.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
|---|
| PCT/JP2008/055765 |

C (Continuation).   DOCUMENTS CONSIDERED TO BE RELEVANT

| Category* | Citation of document, with indication, where appropriate, of the relevant passages | Relevant to claim No. |
|---|---|---|
| A | Kuriyama H. et al., Control of yeast flocculation activity in continuous ethanol fermentation., J. Chem. Eng. Japan, 1993, 26(4), p.429-431 | 1-23 |

Form PCT/ISA/210 (continuation of second sheet) (April 2007)

**REFERENCES CITED IN THE DESCRIPTION**

**Patent documents cited in the description**

- JP 59135896 A